# EUROPEAN PATENT SPECIFICATION

(11) **EP 2 131 687 B1**
(45) Date of publication and mention of the grant of the patent: **28.03.2012**
(21) Application number: 07732949.8
(22) Date of filing: 24.05.2007
(51) Int. Cl.: A41D 19/00, A61B 19/04

(54) **GLOVE WITH ANTI-ROLL DOWN/ANTI-RUCKING CUFF AND CORRESPONDING MANUFACTURING METHOD**
HANDSCHUH MIT NICHT HERUNTERROLLENDER UND KEINE BEULEN BILDENDER STULPE UND ENTSPRECHENDES HERSTELLUNGSVERFAHREN
GANT À MANCHETTE ANTI-ENROULEMENT ET ANTI-FRONCES, ET PROCÉDÉ DE FABRICATION ASSOCIÉ

(30) Priority: 05.09.2006 GB 0617447; 19.10.2006 GB 0620851
(43) Date of publication of application: 16.12.2009
(73) Proprietor: Regent Medical Limited, Irlam, Manchester M44 5BJ (GB)
(72) Inventor: DAY, Jonathan, Lancashire PR7 7BA (GB)
(74) Representative: Hedges, Martin Nicholas
(86) International application number: PCT/GB2007/001929
(87) International publication number: WO 2008/029074

(56) References cited:
- EP-A- 0 359 152
- WO-A-00/76332
- WO-A-2005/102086
- WO-A-2006/100422
- WO-A-2006/107377
- US-A- 3 813 695
- US-A- 6 016 570
- US-A1- 2003 136 410

## Description

The present invention relates to surgical gloves and the like and in particular to such gloves including anti roll down anti-rucking measures provided on the cuff of the glove.

Surgical gloves are designed with a smooth / slippery inner surface with the aim of making it easier for the wearer to don the glove. The gloves are designed to be worn over surgical gowns which were typically cotton based, but recently there have been significant advancements in the material for such gowns, including improvement in the impermeability of the materials achieved by treating the material with a hydrophobic moiety that improves the moisture repellent characteristics of the material. The materials from which surgical gloves are typically made have a tacky, adhesive quality, and in order to facilitate the fitting and removal of the gloves onto and from the hands of the user, the formed glove is treated or coated with a material which reduces the tack of or detackifies the surface thereof. However, an ongoing problem with existing glove and these newer gown designs is that the slippery surfaces of the gloves can give rise to a tendency for the cuff of the glove to move down the gown during use, exposing the woven cuff of the gown and hence presenting a risk of strike through of potentially infectious material.

In order to overcome this problem, gloves have been designed having textured wrist portions which are moulded into the material of the glove during the glove moulding process. For example, US-A-4095293 discloses a moulded glove having a plurality of longitudinal channels moulded around the circumference of the wrist portion of the glove together with a plurality of circumferential grooves in the region of the mouth of the glove which is intended to operate to prevent roll down. However, this arrangement has the problem that the moulding of the grooves into the glove can cause a thinning of material around the wrist and cuff.

Applicants own earlier non-pre-published British patent application no. 0603438.3 discloses the use of masking material applied to an unfinished region of a glove cuff in order to mask the underlying material from the finishing operation and therefore maintain the tacky nature thereof. Subsequent removal of the masking material exposes the underlying tacky glove material which can then provide enhanced friction in contact with the sleeve of a gown.

EP 0359152, upon which the precharacterising portion of claim 1 is based discloses a glove comprising a hand portion and a wrist portion extending from the hand portion and terminating in a hand insertion opening, the wrist portion including a region of friction enhancing material applied to a surface of the wrist portion which, in use, will form the inside of the glove, the friction enhancing material having a degree of tack such that, in use, it will stick to the sleeve of a gown to prevent rolldown/rucking of the glove, whereby, in use, the friction enhancing portion may contact the sleeve of a garment worn by the user so as to adhere the wrist portion of the glove thereto, thereby preventing roll-down/rucking of the glove.

According to the present invention there is provided a glove comprising a hand portion and a wrist portion extending from the hand portion and terminating in a hand insertion opening, the wrist portion including a region of friction enhancing material applied to a surface of the wrist portion which, in use, will form the inside of the glove, the friction enhancing material having a degree of tack such that, in use, it will stick to the sleeve of a gown to prevent roll-down/rucking of the glove, whereby, in use, the friction enhancing portion may contact the sleeve of a garment worn by the user so as to adhere the wrist portion of the glove thereto, thereby preventing rolldown/rucking of the glove; wherein the degree of tack of the friction enhancing material being substantially unaffected by exposure of the friction enhancing material to a finishing operation performed on the glove in order to reduce its tackiness.

A glove in accordance with the invention has the advantage that it provides an effective and reliable system for preventing roll down or rucking of the cuff portion of surgical gloves in a manner which also provides an additional barrier to prevent the ingress of matter between the cuff portion of the glove and the outer surface of the surgical gown.

Preferably, though not essentially, the friction enhancing material extends around the inner circumference of the cuff portion of the glove which may be in a band, so as, in use, to secure the glove to the sleeve of the garment around the entire periphery thereof. This has the advantage of anchoring the glove to the garment in a particularly secure and effective manner. However, the friction material may extend only partially around the cuff portion or may be applied as a discontinuous or broken band around the cuff portion.

The friction enhancing material may alternatively be a pressure sensitive material that displays a surface of moderate tack or no tack prior to its activation. Stimuli that result in activation of the material may include, albeit not exclusively, heat, gamma irradiation, specific wavelengths of light or exposure to certain chemicals which are present in the form of a gas. The material may also be microencapsulated. Once the glove is donned over the gown, the user applies pressure around the cuff area, which causes activation of the material, thereby rendering the cuff area tacky to an extent that the static and dynamic coefficient of friction at the glove-gown interface is increased i.e. providing friction enhancement with the gown.

Preferably, the friction enhancing material is polychloroprene, but other materials are also possible.

Additionally if required the friction enhancing material itself may be overlaid with a specific liner or release material, following the finishing operation, which protects the friction enhancing material, preventing it from sticking until required and from becoming polluted with dust, dirt etc, which might otherwise diminish its effectiveness, until ready for use whilst having a low adhesion to the friction enhancing material so as to be easily removable therefrom. The liner is then removed by the user once the glove has been fitted so as to expose the friction enhancing material for fastening to the sleeve of the gown. The liner material advantageously has identical or similar elastomeric properties to those of the glove so that it expands and contracts with the glove when stretched.

The friction enhancing material strip is formed of material which is separate from the glove material and applied to the surface of the glove at a suitable point after it has been formed, and possibly after a coating process whereby a suitable slip coating is applied to the glove before or after it has been fully dried or cured on line. The glove may or may not be then subjected to a finishing process with a suitable finishing material. The friction enhancing material may optionally be applied as either a solution, a gel, or in the molten state, with liner material or the like applied thereover as required to protect the friction enhancing material until ready for use.

In a particularly preferred embodiment, the friction enhancing material is spray applied using techniques familiar to those skilled in the art, and is of such formulation and suitable viscosity such that it is not destabilised and does not coagulate during spray application.

The present invention further provides a glove and gown assembly comprising a gown having an arm portion terminating in a cuff and a glove according to the invention as claimed, whereby, in use, the friction enhancing portion may contact the sleeve of the gown so as to adhere the wrist portion of the glove thereto, thereby preventing roll-down/rucking of the glove.

The present invention still further provides a method of manufacture of a surgical glove comprising the steps of coating a former with material to form the glove, applying a friction enhancing material to the surface of the glove in the region of a wrist portion thereof, and carrying out a finishing operation the glove to reduce its tackiness; wherein the friction enhancing characteristic of the friction enhancing material being unaffected by exposure of the friction enhancing material to the finishing operation.

Application of the friction enhancing material preferably takes place onto the wrist portion of the glove that has not been subjected to a finishing operation that produces a smooth surface thereof.

The method may also include the step of using of a 'mask' to control and confine the application of friction enhancing material to discrete areas of the glove surface, with the masking material being optionally a suitably absorbent material so as to minimise the opportunity for the friction enhancing material spray to re-bound and disturb the air flow / fluid flow from the spray gun.

In a further advantageous development the friction enhancing material is chilled prior to application to avoid evaporation and the formation of a surface film prior to application.

In order that the invention may be well understood, there will now be described some embodiments thereof.

A surgical glove according to the invention comprises integrally formed hand and cuff portions, the cuff portion terminating in a hand opening by means of which a user may insert a hand into the glove. The glove is manufactured according to normal procedures familiar to those experienced in the art using a former or mould on outer surface of which is formed a film of elastomeric material, such as latex, by straight or coagulant dipping, that is by dipping the former into a solution, after which the former is subsequently dipped into natural or synthetic latices, causing a reaction which results in coagulation of the material, forming a film on the former surface. The film is then dried before being peeled from the mould.

Provided on the cuff portion of the glove is a thin band of friction enhancing material, preferably polychloroprene (Neoprene - RTM), which extends around the circumference of the cuff, substantially parallel and proximate to the hand opening. Some, but not complete drying of the material, which is preferably latex, takes place before application of the polychloroprene, which is preferably applied using a specialised spray process. The polychloroprene applied by the spray process may form a film that is continuous or discontinuous in nature. The polychloroprene is preferably applied in a band of width of 25-45 mm which is 0 - 10 mm from the cuff opening (bead) of the glove. However, it is possible that the polychloroprene could be applied over a much larger area and could be sprayed over the entirety of the glove at this stage.

The polychloroprene is separate to the material(s) of the glove and has a tacky quality, at least with respect to the material(s) of a sleeve of a gown with which it is intended to be used, that tacky quality being substantially unaffected by exposure of the polychloroprene to a finishing operation applied to the material of the glove to reduce its level of tackiness and any other finishing operations applied to the material of the glove for any other purpose.

Following application of the polychloroprene, a drying process may be used to dry the polychloroprene, after which the former may be dipped into a polymer coating that facilitates 'easy-donning' of the glove during use. More particularly, the glove may be dipped so that a region of the glove extending from the cuff opening is left un-coated by the polymer. More particularly, the glove may be dipped so that the polymer coating overlaps the polychloroprene material to a small degree, in particular only about the first 1 - 10% of the area of the band of polychloroprene material, the effective area of the polychloroprene material being left uncoated. This is the only stage in the manufacturing process where care is taken to ensure that the area on the glove to which polychloroprene has been applied remains, to a greater extent, uncoated or untreated.

The glove may be then subjected to manufacturing processes which are known to those skilled in the art, that is drying via heat treatment and subsequent leaching operations. The glove is then removed from the former before further processing may be performed on the glove eg chemical treatment processes to both control and modify the surface grip of the film, to modify the donning characteristics and to reduce residual chemical and particulate levels in or on the resultant glove. These finishing operations are performed on the whole glove, and include a suitable slip coating being applied to the whole glove, the surfaces of the glove being treated, for example chemically and / or with a non powdered material the result of which produces a smooth finish thereon. The adhesiveness of the friction enhancing material (polychloroprene) is, however, unaffected by this finishing operation so that its adhesion to the sleeve of a gown is maintained.

Following the finishing operation, the friction enhancing material may if required be overlaid with a release material so as to protect it from dust and dirt or the like until it is ready for use.

When the glove is ready for use, the user fits the glove onto the hand with the friction enhancing material on the inner surface and the cuff portion positioned to overlap the outer surface of the sleeve of the gown worn by the user. The friction enhancing material in the cuff region then acts so as to increase the static and dynamic coefficient of friction at the interface between the glove and gown. This results in the cuff of the glove being secured on the outer surface of the sleeve of the gown in a manner that prevents roll-down/rucking thereof.

In an alternative embodiment, the polychloroprene may be applied at a later stage in the manufacturing process than in the previous embodiment, in particular following dipping in the polymer that enables 'easy-donning', rather than prior to dipping in this polymer. This would result in there being no overlap between the polychloroprene coating and the donning aid polymer.

## Claims

1. A glove comprising a hand portion and a wrist portion extending from the hand portion and terminating in a hand insertion opening, the wrist portion including a region of friction enhancing material applied to a surface of the wrist portion which, in use, will form the inside of the glove, the friction enhancing material having a degree of tack such that, in use, it will stick to the sleeve of a gown to prevent rolldown/rucking of the glove, whereby, in use, the friction enhancing portion may contact the sleeve of a garment worn by the user so as to adhere the wrist portion of the glove thereto, thereby preventing roll-down/rucking of the glove; **characterised in that** the degree of tack of the friction enhancing material being substantially unaffected by exposure of the friction enhancing material to a finishing operation performed on the glove in order to reduce its tackiness,

2. A glove according to claim 1 which is a surgical glove.

3. A glove according to claim 1 or claim 2, wherein the region of friction enhancing material is applied in a hand having a width of 25 - 45 mm which is 0 - 10 mm from the cuff opening of the glove.

4. A glove according to any of the preceding claims, wherein the region of friction enhancing material extends entirely around the inner circumference of the wrist portion of the glove, and/or wherein the friction enhancing material is a pressure sensitive material which is activated, in use, by application of pressure to the surface thereof.

5. A glove according to any of the preceding claims, wherein a strip of material, in particular release material, overlies the region of friction enhancing material so as to protect it from being polluted after manufacturing but prior to use.

6. A glove according to claim 5, wherein the strip of overlay material has elastomeric properties which are substantially the same as the elastomeric properties of the materials of the glove such that the strip of material stretches and contracts with the underlying glove materials.

7. A glove according to any of the preceding claims, wherein a polymer coating is applied over a majority of the surface of the glove which facilitates easy donning of the glove, the polymer coating extending up to the friction enhancing material, at least part of the friction enhancing material being uncoated by the polymer coating.

8. A glove according to claim 7, wherein the polymer coating partially overlaps the friction enhancing material, by in particular the first 1 - 10% of the width of the friction enhancing material, or wherein none of the friction enhancing material is coated by the polymer coating.

9. A glove and gown assembly comprising a gown having an arm portion terminating in a cuff and a glove according to any of the preceding claims, whereby, in use, the friction enhancing portion may contact the sleeve of the gown so as to adhere the wrist portion of the glove thereto, thereby preventing roll-down/rucking of the glove.

10. A method of manufacture of a surgical glove according to claim 2 or claims 3 to 8 when dependent on claim 2
comprising the steps of coating a former with material to form the glove, applying a friction enhancing material to the surface of the glove in the region of a wrist portion thereof, and carrying out a finishing operation on the glove to reduce its tackiness; **characterised by** the friction enhancing characteristic of the friction enhancing material being unaffected by exposure of the friction enhancing material to the finishing operation.

11. A method according to claim 10, including the further step of partially coating the glove in a polymer that enables easy-donning prior to carrying out the finishing operation.

12. A method according to claim 11, wherein the partial coating is carried out after application of the friction enhancing material, at least part of the friction enhancing material not being coated by the polymer.

13. A method according to claim 11, wherein the partial coating is carried out prior to application of the friction material, the polymer material extending all the way to the friction enhancing material.

14. A method according to any of claims 10 to 13, comprising the further step of applying a removable piece of inner material over the friction enhancing material to protect the friction enhancing material from pollution until ready for use.

## Patentansprüche

1. Handschuh, der einen Handabschnitt und einen Handgelenkabschnitt, der sich von dem Handabschnitt erstreckt und in einer Handeinführungsöffnung endet, umfasst, wobei der Handgelenkabschnitt einen Bereich aus reibungserhöhendem Material aufweist, der auf eine Oberfläche des Handgelenkabschnitts aufgebracht ist und der im Gebrauch die Innenseite des Handschuhs bilden wird, wobei das reibungserhöhende Material einen derartigen Klebrigkeitsgrad hat, dass es im Gebrauch am Ärmel eines Kittels kleben wird, um ein Herunterrollen/Faltenwerfen des Handschuhs zu verhindern, wobei der reibungserhöhende Abschnitt im Gebrauch den Ärmel eines von dem Benutzer getragenen Kleidungsstücks berühren kann, um den Handgelenkabschnitt des Handschuhs daran anhaften zu lassen, wodurch ein Herunterrollen/Faltenwerfen des Handschuhs verhindert wird, **dadurch gekennzeichnet, dass** der Klebrigkeitsgrad des reibungserhöhenden Materials von dem Unterziehen des reibungserhöhenden Materials eines Veredelungsvorgangs, der an dem Handschuh durchgeführt wird, um dessen Klebrigkeit zu verringern, im Wesentlichen nicht beeinträchtigt wird.

2. Handschuh nach Anspruch 1, bei dem es sich um einen Operationshandschuh handelt.

3. Handschuh nach Anspruch 1 oder 2, wobei der Bereich aus reibungserhöhendem Material in einer Hand mit einer Breite von 25 - 45 mm angewendet wird, die sich 0 - 10 mm von der Stulpenöffnung des Handschuhs befindet.

4. Handschuh nach einem der vorhergehenden Ansprüche, wobei der Bereich aus reibungserhöhendem Material sich vollständig um den Innenumfang des Handgelenkabschnitts des Handschuhs erstreckt und/oder wobei das reibungserhöhende Material ein Haftklebematerial ist, das im Gebrauch durch Anwenden von Druck auf dessen Oberfläche aktiviert wird.

5. Handschuh nach einem der vorhergehenden Ansprüche, wobei ein Streifen aus Material, insbesondere Trennmaterial, auf dem Bereich aus reibungserhöhendem Material aufliegt, um diesen vor einer Verschmutzung nach der Herstellung, jedoch vor dem Gebrauch zu schützen.

6. Handschuh nach Anspruch 5, wobei der Streifen aus Auflagematerial elastomere Eigenschaften hat, die im Wesentlichen identisch zu den elastomeren Eigenschaften der Materialien des Handschuhs sind, so dass der Materialstreifen sich mit den darunter liegenden Handschuhmaterialien dehnt und zusammenzieht.

7. Handschuh nach einem der vorhergehenden Ansprüche, wobei eine Polymerbeschichtung über einen Großteil der Oberfläche des Handschuhs aufgebracht wird, was das einfache Anziehen des Handschuhs erleichtert, wobei die Polymerbeschichtung sich bis zu dem reibungserhöhenden Material erstreckt, wobei mindestens ein Teil des reibungserhöhenden Materials nicht mit der Polymerbeschichtung beschichtet wird.

8. Handschuh nach Anspruch 7, wobei die Polymerbeschichtung teilweise mit dem reibungserhöhenden Material überlappt, um insbesondere die ersten 1 - 10 % der Breite des reibungserhöhenden Materials, oder wobei überhaupt kein Teil des reibungserhöhenden Materials mit der Polymerbeschichtung beschichtet ist.

9. Handschuh- und Kittelkombination, die einen Kittel mit einem Armabschnitt, der in einem Stulpen endet, und einen Handschuh nach einem der vorhergehenden Ansprüche umfasst, wobei der reibungserhöhende Abschnitt im Gebrauch den Ärmel des Kittels berühren kann, um den Handgelenkabschnitt des Handschuhs daran anhaften zu lassen, wodurch ein Herunterrollen/Faltenwerfen des Handschuhs verhindert wird.

10. Verfahren zur Herstellung eines Operationshandschuhs nach Anspruch 2 oder den Ansprüchen 3 bis 8 bei Abhängigkeit von Anspruch 2, wobei das Verfahren die Schritte des Beschichtens eines ersteren mit einem Material, um den Handschuh zu bilden, des Aufbringens eines reibungserhöhenden Materials auf die Oberfläche des Handschuhs im Bereich eines Handgelenkabschnitts davon und des Ausführens eines Veredelungsvorgangs an dem Handschuh, um dessen Klebrigkeit zu verringern, umfasst; **dadurch gekennzeichnet, dass** der reibungserhöhende Charakter des reibungserhöhenden Materials von dem Unterziehen des reibungserhöhenden Materials des Veredelungsvorgangs nicht beeinträchtigt wird.

11. Verfahren nach Anspruch 10, das den weiteren Schritt des partiellen Beschichtens des Handschuhs in einem Polymer, das ein einfaches Anziehen ermöglicht, vor dem Ausführen des Veredelungsvorgangs aufweist.

12. Verfahren nach Anspruch 11, wobei das partielle Beschichten nach dem Aufbringen des reibungserhöhenden Materials ausgeführt wird, wobei mindestens ein Teil des reibungserhöhenden Materials nicht mit dem Polymer beschichtet wird.

13. Verfahren nach Anspruch 11, wobei das partielle Beschichten vor dem Aufbringen des reibungserhöhenden Materials ausgeführt wird, wobei das Polymermaterial sich ganz bis zu dem reibungserhöhenden Material erstreckt.

14. Verfahren nach einem der Ansprüche 10 bis 13, das den weiteren Schritt des Aufbringens eines abnehmbaren Stücks aus Innenmaterial über dem reibungserhöhenden Material umfasst, um das reibungserhöhende Material vor einer Verschmutzung zu schützen, bis es gebrauchsfertig ist.

## Revendications

1. Gant comportant une partie main et une partie poignet se prolongeant depuis la partie main et se terminant en une ouverture d'insertion de la main, la partie poignet comprenant une région composée d'un matériau augmentant la friction appliquée sur une surface de la partie poignet qui, à l'utilisation, formera l'intérieur du gant, le matériau augmentant la friction ayant un pouvoir adhérant tel qu'à l'utilisation, il adhérera à la manche du vêtement porté par l'utilisateur afin d'empêcher le roulement vers le bas/la fronce du gant si bien qu'à l'utilisation, la partie augmentant la friction peut être en contact avec la manche du vêtement porté par l'utilisateur de façon à ce que la partie poignet du gant y adhère, empêchant ainsi le roulement vers le bas/la fronce du gant ; **caractérisé en ce que** le pouvoir adhérant du matériau augmentant la friction n'est pas sensiblement affecté par une exposition du dit matériau augmentant la friction à une opération de finition effectuée sur le gant afin de réduire son adhésivité.

2. Gant selon la revendication 1, qui est un gant chirurgical.

3. Gant selon la revendication 1 ou la revendication 2, où la région de matériau augmentant la friction est appliquée sur une portion d'un gant de 25 - 45 mm de large située à 0 - 10 mm de la manchette ouvrant le gant.

4. Gant selon l'une quelconque des revendications précédentes, où la région de matériau augmentant la friction s'étend sur la totalité de la circonférence interne de la partie poignet du gant et/ou où le matériau augmentant la friction est un matériau sensible à la pression qui est activé, à l'utilisation, par l'application d'une pression à la surface de celui-ci.

5. Gant selon l'une quelconque des revendications précédentes, où une bande d'un matériau, en particulier d'un matériau de dégagement, recouvre la région de matériau augmentant la friction de façon à la protéger contre la pollution après la fabrication mais avant l'utilisation.

6. Gant selon la revendication 5, où la bande de matériau de recouvrement a des propriétés élastomères qui sont essentiellement les mêmes que les propriétés élastomères des matériaux du gant, si bien que la bande de matériau s'étire et se contracte avec les matériaux sous-jacents du gant.

7. Gant selon l'une quelconque des revendications précédentes, où un revêtement polymère est appliqué sur une majorité de la surface du gant pour favoriser l'enfilement facile du gant, le revêtement polymère s'étendant jusqu'au matériau augmentant la friction et une portion au moins du matériau augmentant la friction n'étant pas recouverte par le revêtement polymère.

8. Gant selon la revendication 7, où le revêtement polymère chevauche en partie le matériau augmentant la friction, en particulier les premiers 1 - 10 % de la largeur du matériau augmentant la friction, ou où aucune portion du matériau augmentant la friction n'est recouverte par le revêtement polymère.

9. Assemblage gant-blouse de chirurgie comprenant une blouse de chirurgie dont une portion de la manche se termine par un poignet et un gant selon l'une quelconque des revendications précédentes où, à l'utilisation, la portion augmentant la friction peut être en contact avec la manche de la blouse de façon à ce que la partie poignet du gant y adhère, empêchant ainsi le roulement vers le bas/la fronce du gant.

10. Méthode de fabrication d'un gant chirurgical selon la revendication 2 ou les revendications 3 à 8 quand dépendantes de la revendication 2, comprenant les étapes de revêtement d'un gabarit avec un matériau pour former le gant, l'application d'un matériau augmentant la friction à la surface du gant dans une région de la partie poignet de celui-ci et l'exposition du gant à une opération de finition dans le but de réduire son adhésivité, **caractérisée en ce que** les caractéristiques augmentant la friction du matériau augmentant la friction ne sont pas affectées par l'exposition du matériau augmentant la friction à l'opération de finition.

11. Méthode selon la revendication 10, qui comprend l'étape supplémentaire consistant à revêtir en partie le gant avec un polymère qui permet son enfilement facile avant d'effectuer l'opération de finition.

12. Méthode selon la revendication 11, où le revêtement partiel est effectué après l'application du matériau augmentant la friction, une portion au moins du matériau augmentant la friction n'étant pas recouverte par le revêtement polymère.

13. Méthode selon la revendication 11, où le revêtement partiel est effectué avant l'application du matériau augmentant la friction, le revêtement polymère s'étendant jusqu'au matériau augmentant la friction.

14. Méthode selon l'une quelconque des revendications 10 à 13, qui comprend l'étape supplémentaire consistant à appliquer un fragment détachable d'un matériau interne sur le matériau augmentant la friction de façon à protéger le matériau augmentant la friction contre la pollution jusqu'à ce qu'il soit prêt à être utilisé.
